(19) Europäisches Patentamt
European Patent Office
Office européen des brevets

(11) **EP 3 241 489 A1**

(12) **EUROPEAN PATENT APPLICATION**

(43) Date of publication:
**08.11.2017 Bulletin 2017/45**

(51) Int Cl.:
**A61B 5/00** (2006.01)  **A61B 5/0476** (2006.01)
**A61B 5/0482** (2006.01)  A61B 5/048 (2006.01)

(21) Application number: **16168344.6**

(22) Date of filing: **04.05.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Mensia Technologies**
**35571 Chantepie Cedex (FR)**

(72) Inventors:
• OJEDA, David
75014 Paris (FR)
• BARTHELEMY, Quentin
92170 Vanves (FR)
• MAYAUD, Louis
75020 Paris (FR)

(74) Representative: **Icosa**
**83 avenue Denfert-Rochereau**
**75014 Paris (FR)**

(54) **PREDICTIVE NEUROMARKERS OF ALZHEIMER'S DISEASE**

(57)    The present invention relates to predictive neuromarkers of Alzheimer's disease comprising at least one spectral feature obtained from EEG signals of a subject; and at least one Riemannian distance between a spatiofrequential covariance matrix computed from the EEG signals of said subject and at least one reference spatiofrequential covariance matrix. The present invention also relates to a non-invasive method of diagnosing the presence of Alzheimer's disease in a subject using the predictive neuromarkers of Alzheimer's disease; and to a method for self-paced modulation of EEG signals of a subject in order to alleviate symptoms of Alzheimer's disease using the predictive neuromarkers of Alzheimer's disease.

FIG. 2

EP 3 241 489 A1

**Description**

**FIELD OF INVENTION**

The present invention pertains to the field of assessment, diagnosis, and treatment of a medical condition. More specifically, the present invention relates to predictive neuromarkers of Alzheimer's disease (AD). The present invention also relates to a non-invasive method of diagnosing the presence of AD using said predictive neuromarkers and to a neurofeedback method to alleviate symptoms of AD using said predictive neuromarkers.

**BACKGROUND OF INVENTION**

AD is a neurodegenerative disorder during which neural tissue is gradually degraded leading to progressive loss of intellectual, behavioral and functional abilities. AD is the leading cause of dementia in humans and already one of the most important financial burden for society. AD diagnosis is currently performed based upon clinical history, laboratory tests, neuroimaging and neuropsychological evaluations. However theses clinical assessments are costly and require experiences clinicians and/or lengthy sessions.

As the therapies to treat AD are still not effective, possibly due to irreversible brain damages, there is a need for an accurate, specific and cost effective biomarker to assess and diagnose AD at an early stage to reduce the risk of a later development. There is also a need of a biomarker to follow disease progression and therapy response.

The symptoms of AD have been reported to be associated with changes in the cortical electrical activity recorded by electroencephalography (EEG). Neuromarkers identification is then a major concern as it could improve the early diagnostic of the disease, and enable brain waves training (neurofeedback training) in order to correct EEGs anomalies and reduce cognitive impairments.

Several studies have already reported EEG modifications in AD patient's brain (Bhat et al., 2015, Clinical Neurophysiological and Automated EEG-Based Diagnosis of the Alzheimer's Disease, Eur Neurol, DOI: 10.1159/000441447). Spectral measures report a global slowing of brain activity with power increase in delta and theta rhythms; a power decrease in alpha and/or beta rhythms mostly in frontal-central and parietal regions; and an elevated activity in gamma band in parietal, occipital and posterior temporal regions (Vialatte & Gallego, 2014, A Theta-band EEG based Index for Early diagnosis of Alzheimer's disease, Journal of Alzheimer's disease, DOI 10.3233/JAD-140468; Lizio et al., 2011, Electroencephalographic Rhythms in Alzheimer's Disease, International Journal of Alzheimer's Disease, Vol. 2011, DOI 10.4061/2011/927573; Deursen et al., 2008, Increased EEG gamma band activity in Alzheimer's disease and mild cognitive impairment, J Neural Transm, DOI 10.1007/s00702-008-0083-y).

It is an object of the invention to provide predictive EEG features that relates to the risk of Alzheimer's disease in patients with improved sensibility, specificity, accuracy and/or AUROC with regards to known spectral analysis.

It is a further object of the invention to use said predictive neuromarkers in diagnosis of AD and in a neurofeedback method to alleviate the symptoms of AD.

**DEFINITIONS**

In the present invention, the following terms have the following meanings:

- **"About"** preceding a figure means plus or less 10% of the value of said figure, preferably plus or less 5% of the value of said figure.

- **"AUROC"** stands for area under the ROC curve, and is an indicator of the accuracy of a diagnostic test. In statistics, a receiver operating characteristic (ROC), or ROC curve, is a graphical plot that illustrates the performance of a binary classifier system as its discrimination threshold is varied. The curve is created by plotting the sensitivity against the specificity (usually 1- specificity) at successive values from 0 to 1.

- **"Biomarker"** refers to a variable that may be measured from a bodily fluid sample, such as for example a blood or cerebrospinal fluid sample, or from medical imaging techniques, such as for example positron emitting tomography (PET) scan, magnetic resonance imagery (MRI), computed tomography (CT) scan, retina scan, or from any other diagnosis tool.

- **"Computing device"** refers to a computer-based system or a processor-containing system or other system that can fetch and execute the instructions of a computer program.

- **"Diagnostic cut-off"** refers to the diagnostic cut-off of a non-invasive test score. The cut-off distinguishes patients with or without the diagnostic target (yes/no). Within a preferred embodiment of the present invention, the diagnostic cut-off is the threshold that minimized the distance to the top-left corner of the ROC plot. The diagnostic cut-off may also be fixed a priori to 0.5 according to statistical convention, and a posteriori according to specific choice, usually the highest Youden index (Se+Spe-1), the maximum overall accuracy to optimize test performance or the threshold with the highest sensitivity and specificity.

- **"Diagnostic target"** refers to the main objective of a non-invasive diagnostic test, i.e. for determining the presence or absence (yes/no) of a targeted clinical feature. Thus, the diagnostic target of the present invention is the presence or absence of AD.

- **"Electrode"** refers to a conductor used to establish electrical contact with a nonmetallic part of a circuit. For instance EEG electrodes are small metal discs usually made of stainless steel, tin, gold, silver covered with a silver chloride coating; there are placed on the scalp in specific positions.

- **"Epoch"** refers to a determined period over which EEG signals are analyzed.

- **"External or induced modulation"** refers to the modulation of the brain activity which is not induced by the subject. Said modulation may comprise the following methods:

  - Deep brain stimulation (DBS);
  - Electroconvulsive therapy (ECT);
  - Magnetic seizure therapy (MST);
  - Transcranial direct current stimulation (tDCS);
  - Transcranial magnetic stimulation (TMS);
  - Repetitive transcranial magnetic stimulation (rTMS); or
  - Vagus nerve stimulation (VNS).

External modulation also comprises any method of stimulation known by one skilled in the art which affect the brain's activity, e.g. drugs (sedation) or interventions (mechanical ventilation). Such stimulation may also indirectly affect the brain via sensory neural afferences: acoustic, visual, somatosensory stimulations. External modulation may also comprise simultaneous stimulation of elements of the two hemispheres of the brain at different frequencies of phase in order to elicit brain activity at frequency of interest in specific area of the brain (e.g. binaural beats for auditory stimulation).

- **"Metaphor"** refers to a particular mental task to focus on which is associated with producing or achieving a target brain state response in the subject. For instance, for achieving a particular brain state, a metaphor may be a target. The target may come into focus when the subject's brain state is closer to the target brain state, and the target may go out of focus when the subject's brain state is further from the target brain state.

- **"Mild cognitive impairment"** (MCI) is considered as a transitional stage between normal aging and dementia. AD symptoms typically start with MCI. The etiology of MCI is not restricted to AD.

- **"Neuromarker"** refers to a quantitative measure derived from the EEG.

- **"Negative predictive value (NPV)"** refers to the proportion of patients with a negative test result that are actually disease free; if 8 of 10 negative test results are correct (true negative), the NPV is 80%. Because not all negative test results are true negatives, some patients with a negative test result actually have the disease. The NPV describes how likely it is that a negative test result in a given patient population represents a true negative.

- **"Patient"** refers to a subject awaiting the receipt of, or is receiving medical care or is/will be the object of a medical procedure for treating Alzheimer's disease.

- **"Positive predictive value (PPV)"** refers to the proportion of patients with a positive test that actually have disease; if 9 of 10 positive test results are correct (true positive), the PPV is 90%. Because all positive test results have some number of true positives and some false positives, the PPV describes how likely it is that a positive test result in a given patient population represents a true positive.

- **"Predictive neuromarkers"** refers to neuromarkers that may be used to discriminate whether a subject has a particular condition, especially AD.

- **"Real time"** refers to a process for which the output is given within a time delay that is considered as smaller than the time delay required to perform the underlying task of modulation adequately. Therefore for self-paced modulation, real time refers to a process implemented in less than 700ms, preferably less than 500ms, more preferably less than 400ms, even more preferably less than 250ms. For external modulation real time may refer to a process implemented in less than 10min, less than 1min; less than 30s, less than 1s or less than 700ms, depending on the frequency of the external modulation.

- **"Riemannian manifold"** refers to a differentiable topological space that is locally homeomorphic to a Euclidean space, and over which a scalar product is defined that is sufficiently regular. The scalar product makes it possible to define a Riemannian geometry on the Riemannian manifold.

- **"Score"** refers to any digit value obtained by the mathematical combination of at least one neuromarker and/or at least biomarker. In one embodiment, a score is an unbound digit value. In another embodiment, a score is a bound digit value, obtained by a mathematical function. Preferably, a score ranges from 0 to 1.

- **"Self-paced modulation"** refers to the modulation of the brain activity induced by the subject. In the sense of the present invention, self-paced modulation has the same meaning as neurofeedback and refers to the ability for the subject to control its brain electrical activity in real time. Self-paced modulation may include cognitive strategy such as predefined instructions given to the subject.

- **"Sensitivity"** (also called true positive rate) measures the proportion of actual positives which are correctly identified as such.

- **"Specificity"** (also called true negative rate) measures the proportion of negatives which are correctly identified as such.

- **"Subject"** refers to a mammal, preferably a human. In one embodiment, a subject is a "patient", i.e. a warm-blooded animal, more preferably a human, who/which is awaiting the receipt of, or is receiving, medical care or was/is/will be the subject of a medical procedure, or is monitored for the development or progression of a disease.

- **"Symmetric positive definite (SPD) matrix"** refers to a square matrix that is symmetrical about its diagonal (i.e. $A_{ij}=A_{ji}$) and that has eigenvalues that are strictly positive. An SPD matrix of dimensions C*C has C(C+1)/2 independent elements; it may therefore be locally approximated by an Euclidian space of C(C+1)/2 dimensions. It is possible to show the SPD space has the structure of a Riemannian manifold. It is known that covariance matrices are symmetric positive definite matrices.

## DETAILED DESCRIPTION

This invention relates to predictive neuromarkers of Alzheimer's disease.

Said predictive neuromarkers comprise at least one spectral feature obtained from EEG signals of a subject; and at least one Riemannian distance between a spatiofrequential covariance matrix computed from the EEG signals of said subject and at least one reference spatiofrequential covariance matrix.

Various types of suitable headsets or electrode systems are available for acquiring such EEG signals. Examples includes, but are not limited to: Epoc headset commercially available from Emotiv, Mindset headset commercially available from Neurosky, Versus headset commercially available from SenseLabs, DSI 6 headset commercially available from Wearable sensing, Xpress system commercially available from BrainProducts, Mobita system commercially available from TMSi, Porti32 system commercially available from TMSi, ActiChamp system commercially available from BrainProducts and Geodesic system commercially available from EGI.

According to one embodiment, the EEG signals are acquired using a set of sensors and/or electrodes. According to one embodiment, the EEG signals are acquired by at least 4, 8, 10, 15, 16, 17, 18, 19, 20, 25, 50, 75, 100, 150, 200, 250 electrodes.

The overall acquisition time is subdivided into periods, known in the art as epochs. Each epoch is associated with a matrix $X \in \mathbb{R}^{C*N}$, representative of the spatiotemporal signals acquired during said epoch. Spatiotemporal EEG signals $X \in \mathbb{R}^{C*N}$ are composed of C channels, electrodes or sensors and N time samples. For example, a subject is fitted with C electrodes for EEG signals acquisitions. Each electrode c=1...C delivers a signal $X_c(n)$ as a function of time. The signal is sampled so as to operate in discrete time: $X(c, n)=X_c(n)$, and then digitized. This produced a matrix representation of the set of EEG signals. According to one embodiment, in order to ensure real-time processing, successive epochs are overlapped.

According to one embodiment, the covariance matrix is a spatial covariance matrix. In an embodiment, the spatial covariance matrix is computed as follows: $M = \frac{1}{N-1} X^T X$, with $N \geq C$, where N is the number of samples in the epoch for each electrode and C the number of electrodes. In another embodiment, the spatial covariance matrix is computed using any method known by the skilled artisan, such as those disclosed in Barachant A. Commande robuste d'un effecteur par une interface cerveau-machine EEG asynchrone, PhD. Thesis, Université de Grenoble: FR, 2012.

According to one embodiment, the EEG signals are filtered in at least one frequency band, preferably four frequency bands, namely alpha, beta, theta and delta frequency bands.

According to one embodiment, the signal $X \in \mathbb{R}^{C*N}$ is filtered in F frequency bands; thereby obtaining f = 1... F filtered signals $X_f \in \mathbb{R}^{C*N}$. According to one embodiment, the extended signal $\widetilde{X} \in \mathbb{R}^{CF*N}$ is defined as the vertical concatenation of the filtered signals:

$$\widetilde{X} = \begin{bmatrix} X_1 \\ \vdots \\ X_f \\ \vdots \\ X_F \end{bmatrix}.$$

According to one embodiment, the covariance matrix is a spatiofrequential covariance matrix. In one embodiment, the spatiofrequential covariance matrix $M \in \mathbb{R}^{CF*CF}$ is computed as follows: $\widetilde{M} = \frac{1}{N-1} \widetilde{X}^T \widetilde{X}$, with $N \geq CF$ where N is the number of samples in the epoch for each electrode, and C the number of electrodes. According to one embodiment, the spatio-frequential covariance matrix can be normalized, as described hereafter, by its trace or its determinant.

According to one embodiment, the covariance matrix is normalized. According to one embodiment, the covariance matrix is trace-normalized, which makes its trace equal to 1:

$$M = \frac{M}{trace\,(M)}.$$

According to one embodiment, the covariance matrix is determinant-normalized, which makes its determinant equal to 1:

$$M = \frac{M}{det(M)^{1/C}}.$$

According to one embodiment, the EEG signals are pre-processed. According to one embodiment, the EEG signals are centered. According to one embodiment, the EEG signals are resampled. According to one embodiment, the EEG signals are filtered with a band-pass and/or a band-stop filter. According to one embodiment, the EEG signals are filtered with a band-pass and/or a band-stop filter. According to one embodiment, the EEG signals are spatially reconstructed over the international 10-20 system. According to the one embodiment the signals are re-referenced using the common average reference (CAR).

According to one embodiment, after pre-processing, an artefact rejection method is implemented; preferably a Riemannian potato field.

According to embodiment, the at least one spectral feature is selected from the spectral power density for at least one frequency range for at least one electrode.

According to one embodiment, the at least one frequency range is selected from alpha frequency range, beta frequency range, delta frequency range, gamma frequency range and theta frequency range.

According to one embodiment, the at least one electrode is at least one electrode located according to the international 10-20 system.

According to embodiment, the at least one spectral feature is selected from the spectral power density for alpha frequency range for at least one electrode according to the international 10-20 system, the spectral power density for beta frequency range for at least one electrode according to the international 10-20 system, the spectral power density for delta frequency range for at least one electrode according to the international 10-20 system, the spectral power density for gamma frequency range for at least one electrode according to the international 10-20 system and/or the spectral power density for theta frequency range for at least one electrode according to the international 10-20 system.

According to embodiment, the at least one spectral feature is selected from at least one spectral power density for alpha frequency range for at least one electrode according to the international 10-20 system, at least one spectral power density for beta frequency range for at least one electrode according to the international 10-20 system, at least one spectral power density for delta frequency range for at least one electrode according to the international 10-20 system, at least one spectral power density for gamma frequency range for at least one electrode according to the international 10-20 system and/or at least one spectral power density for theta frequency range for at least one electrode according to the international 10-20 system.

According to embodiment, the at least one spectral feature is selected from at least one spectral power density for alpha frequency range for from 1 to 10 electrode according to the international 10-20 system, at least one spectral power density for beta frequency range for from 1 to 10 electrode according to the international 10-20 system, at least one spectral power density for delta frequency range for from 1 to 10 electrode according to the international 10-20 system, at least one spectral power density for gamma frequency range for from 1 to 10 electrodes according to the international 10-20 system and/or at least one spectral power density for theta frequency range for from 1 to 10 electrodes according to the international 10-20 system.

According to embodiment, the at least one spectral feature is selected from the spectral power densities for alpha, beta, theta, gamma and/or delta frequency ranges for electrodes Fp1; Fp2; F7; F3; Fz; F4; F8; T3; C3; Cz; C4; T4; T5; P3; Pz; P4; T6; O1 and/or 02 according to the international 10-20 system. According to embodiment, the at least one spectral comprises at least 60%, at least 50%, at least 40%, at least 30%, at least 20% of the spectral power densities for alpha, beta, theta, gamma and/or delta frequency ranges for electrodes Fp1; Fp2; F7; F3; Fz; F4; F8; T3; C3; Cz; C4; T4; T5; P3; Pz; P4; T6; O1 and/or 02 according to the international 10-20 system.

According to embodiment, the at least one spectral feature is selected from the spectral power densities for alpha frequency range for Fp2, F7, C3, C4, P3 and 02 electrodes; the spectral power densities for theta frequency range for Fp2, F3, F4,F8, Cz, T4, P4 and O1 electrodes, the spectral power densities for beta frequency range for F3, F4, T3, Cz, C4, T4, P3 and P4 electrodes, and the spectral power densities for delta frequency range for F3, F8, Cz, P3, Pz, T6 and O2 electrodes.

According to one embodiment, the at least one spectral features comprises the spectral power density for alpha frequency range for Fp2 electrode; the spectral power density for theta frequency range for P4 electrode and the spectral power density for alpha frequency range for 02 electrode.

Each covariance matrix associated with a given epoch is considered to be a point of a Riemannian manifold.

According to one embodiment, the Riemannian distance between two covariance matrices A and B is defined as the affine-invariant distance:

$$d(A, B) = [\sum_{i=1}^{n} \ln^2 \lambda_i(A, B)]^{\frac{1}{2}},$$

with $\lambda_i(A, B)$ the eigenvalues from $|\lambda A - B| = 0$.

In another embodiment, the Riemannian distance is computed using any other distances known by one skilled in the art, such as those described in Li Y, Wong KM. Riemannian Distances for Signal Classification by Power Spectral Density. IEEE Journal of selected topics in signal processing, vol.7, No.4, August 2013.

According to one embodiment, the Riemannian distances are estimated on the Riemannian manifold of symmetric positive definite matrices of dimensions equal to the dimensions of the covariance matrices.

According to one embodiment, the at least one reference spatiofrequential covariance matrices are obtained by a Riemannian clustering method from spatiofrequential covariance matrices from a database.

According to one embodiment, the Riemannian clustering method is selected from Mean-shift, k-means, average or principal geodesic analysis (PGA).

According to one embodiment, the at least one Riemannian distance of the predictive neuromarkers comprises the Riemannian distance between the spatiofrequential covariance matrix computed from the EEG signals of said subject and at least one reference spatiofrequential covariance matrix characteristics of a population of Alzheimer subjects.

According to one embodiment, the at least one Riemannian distance comprises:

- the Riemannian distance between the spatiofrequential covariance matrix computed from the EEG signals of said subject and at least one reference spatiofrequential covariance matrix characteristics of a population of Alzheimer subjects;

- the Riemannian distance between the spatiofrequential covariance matrix computed from the EEG signals of said subject and a reference spatiofrequential covariance matrix characteristics a control population which does not suffer from Alzheimer's disease or mild cognitive impairment; and
- the Riemannian distance the spatiofrequential covariance matrix computed from the EEG signals of said subject and at least one reference spatiofrequential covariance matrix characteristics of a population of mild cognitive impairment subjects.

According to one embodiment, the at least one reference spatiofrequential covariance matrix characteristics of a population of Alzheimer subjects, the at least one reference spatiofrequential covariance matrix characteristics of a control population and/or the at least one reference spatiofrequential covariance matrix characteristics of a population of mild cognitive impairment subjects is obtained by a Riemannian clustering method from spatiofrequential covariance matrices of EEG signals of respectively a population of Alzheimer subjects, a control population and/or a population of mild cognitive impairment subjects.

The metric used for covariance matrices has been detailed in Förstner W, Moonen B. A metric for covariance matrices. Quo vadis geodesia, pp. 113-128, 1999.

According to one embodiment, the predictive neuromarkers further comprises the signal complexity and/or metrics derived from information theory.

According to one embodiment, the predictive neuromarker is combined with biomarkers; especially biomarkers derived from cerebrospinal fluid (CSF), blood samples, or medical imaging techniques such as positron emitting tomography (PET) scan, magnetic resonance imagery (MRI), computed tomography (CT) scan, retina scan, or any other diagnosis tool.

Combining the predictive neuromarkers with other metric would typically increase the specificity, the sensitivity, or reduce the cost of the prediction by replacing more expensive measurements by EEG with no loss in predictive power.

According to one embodiment, the predictive neuromarkers are selected using a machine learning algorithm that classifies the subjects from the combination of features (spectral, complexity, information theory, and Riemannian). Said machine learning algorithm enables selection of predictive neuromarkers.

According to one embodiment, the selection is performed using a regularized linear model, such as a least absolute shrinkage and selection operator (LASSO) general linear model. According to another embodiment, the selection is performed using random forests, support vector machines or neural networks.

According to one embodiment, the classification is performed using any machine learning algorithm known to one skilled in the art.

The present invention also relates to a non-invasive method of diagnosing the presence of Alzheimer's disease in a subject, comprising:

1) computing the predictive neuromarkers of Alzheimer's disease from EEG signals of said subject according to the present invention; and
2) combining said predictive neuromarkers in a mathematic function to obtain a score useful for diagnosing the presence of Alzheimer's disease in said subject.

According to one embodiment, the non-invasive method of diagnosing the presence of Alzheimer's disease in a subject further comprises the step of diagnosing the presence or absence of Alzheimer's disease in said subject if the score is respectively below of above a diagnostic cut-off.

According to one embodiment, the mathematic function is a logistic function, preferably computed as follows:

$$p(x \in \mathrm{AD} \mid w, w_0) = \frac{1}{1 + \exp(-(x^T w + w_0))},$$

wherein x is a vector of the features (including spectral or Riemannian), w is the vector of the model coefficients and $w_0$ is a bias term. The range of this logistic function is real open interval between 0 and 1 used as a score.

According to one embodiment, the model coefficients are obtained using the machine learning algorithm as described hereabove.

According to one embodiment, the score is combined with other biomarkers.

Combining the EEG derive score with other biomarkers would typically increase the specificity, the sensitivity, or reduce the cost of the prediction by replacing more expensive measurements by EEG with no loss in predictive power.

In one embodiment, the method of the invention is computer implemented.

Thus the invention also relates to a microprocessor to implement a non-invasive method for diagnosing AD in a subject as described hereinabove.

The present invention also relates to a method for self-paced modulation of EEG signals of a subject in order to alleviate symptoms of AD, said method comprising continuously:

- acquiring EEG signals from the subject; and
- computing the predictive neuromarkers of Alzheimer's disease from EEG signals of said subject according to the present invention;
- computing a score from the predictive neuromarker; and
- reporting in real time to the subject the score.

According to one embodiment, the score is computed as described hereabove by combining the predictive neuromarkers in a mathematic function, preferably a logistic regression.

By reporting in real time to the subject a score, the subject is able to control the brain electrical activity such that the score can be manipulated by the subject in real time.

According to one embodiment, instructions are given to the subject during the session of self-paced modulation; said instructions includes, but are not limited to, relax, breathe normally, remain quiet, avoid eye movement, avoid muscle tension, avoid sucking movements, avoid chewing, or avoid any movement.

According to one embodiment, no instructions are given to the subject during the session of self-paced modulation.

The present invention also relates a method for external modulation of EEG signals of a subject in order to alleviate symptoms of AD, said method comprising:

- acquiring EEG signals from the subject;
- computing the predictive neuromarkers of Alzheimer's disease from EEG signals of said subject according to the present invention; and
- reporting in real time to an operator a score obtained by the method according to the present invention;
- applying external modulation to the subject in order to modulate the score.

According to one embodiment, the score is computed as described hereabove by combining the predictive neuromarkers in a mathematic function, preferably a logistic regression.

According to one embodiment, the method for external modulation of EEG signals of a subject is not therapeutic.

According to one embodiment, the external modulation is applied by indirect brain stimulation, deep brain stimulation (DBS), electroconvulsive therapy (ECT), magnetic seizure therapy (MST), transcranial direct current stimulation (tDCS), transcranial magnetic stimulation (TMS), repetitive transcranial magnetic stimulation (rTMS) or Vagus nerve stimulation (VNS). According to one embodiment, the external modulation comprises indirect brain stimulation such as any sensory stimulation (auditory, visual, somatosensory).

The present invention also relates a system for self-paced modulation or external modulation of EEG signals of a subject comprising:

- acquisition means for acquiring EEG signals from a subject;
- computing device for computing the predictive neuromarkers of Alzheimer's disease from EEG signals of said subject according to the present invention and for computing a score from the predictive neuromarkers; and
- output means for reporting the score to the subject using a metaphor.

According to one embodiment, the acquisition means comprises any means known by one skilled in the art enabling acquisition (i.e. capture, record and/or transmission) of EEG signals as defined in the present invention, preferably electrodes or headset as explained hereabove. According to one embodiment, the acquisition means comprises an amplifier unit for magnifying and/or converting the EEG signals from analog to digital format.

According to one embodiment, the computing device comprises a processor and a software program. The processor receives digitalized EEG signals and processes the digitalized EEG signals under the instructions of the software program to compute the score. According to one embodiment, the computing device comprises memory.

According to one embodiment, the computing device comprises a network connection enabling remote implementation of the method according to the present invention. According to one embodiment, EEG signals are communicated to the computing device. According to one embodiment, the output means receives the score from the computing device.

According to one embodiment, the output means comprise any means for reported the score. According to one embodiment, the score is reported using anyone of the senses of the subject: visual means, auditory means, olfactory means, tactile means (e.g. vibratory or haptic feedback) and/or gustatory means. Preferably the score is reported using a display such as a screen: a smartphone, a computer monitor or a television; or a head-mounted display.

According to one embodiment, especially in the case of self-paced modulation, the reporting of the score enables the subject to be aware of the right direction of the training. According to one embodiment, the reporting of the score comprises a visual reporting wherein a target, representing the real-time score of the subject, is displayed, said target moving towards or away from a location representing a target score defined for instance by a non-AD state.

According to one embodiment wherein the score is reported using auditory means, a sound, the amplitude of which is directly modulated by said score, is reported to the subject. The sound can be a simple beep, water flowing, waves, rain, dongs, or any other sound which can be modulated in amplitude or frequency.

According to one embodiment wherein the score is reported using visual means, an object on the screen, which position, size, color, or any other parameters can be modulated by said score, is reported to the subject. For instance it can be the representation of a plane, the altitude of which is modulated by the score.

The present invention also relates to a method for monitoring a patient, wherein said method comprises implementing at time intervals the non-invasive method of the invention, thereby assessing the evolution of said patient by comparing the values of the scores obtained at time intervals by the patient.

The present invention also relates to a tool for helping in medical decisions regarding a patient suffering from AD, wherein said method comprises (i) implementing the non-invasive method of the invention and (ii) selecting in a database pharmaceutical compositions which could be suitable for the patient according to the value of the score obtained by the patient.

In one embodiment, the method of the invention is implemented before the administration of a treatment to a patient and at least once during or after the administration of a treatment to said patient. In another embodiment, the method of the invention is implemented before the administration of a treatment to a patient and at regular time intervals during the administration of a treatment to said patient. Said embodiments enable to follow the efficacy of a treatment or to improve its design during a development or clinical research phase or for its titration during home delivery

While various embodiments have been described, the detailed description is not to be construed as being limited hereto. Various modifications can be made to the embodiments by those skilled in the art without departing from the true spirit and scope of the disclosure as defined by the claims.

## BRIEF DESCRIPTION OF THE DRAWINGS

**Figure 1** illustrates a spatiofrequential covariance matrix in the frequency bands alpha, beta, theta and delta.

**Figure 2** illustrates the location of selected variables (electrodes) for every frequency range.

**Figure 3** illustrates ROC curves and optimal cutoff point of models for each database fold.

## EXAMPLES

The present invention is further illustrated by the following study.

### *Database*

Study was conducted on international independent dataset, collected in AD, MCI, and control subjects. All signals were acquired under eyes closed (EC) condition. The input data is highly heterogeneous in terms of protocol, number of channels, and sampling rate, as detailed in Table 1 hereafter. Patients in different groups were not necessarily age or gender matched.

**Table 1: Description of different databases used for the study**

| Location / database | Subjects | Number of EEG channels | Signal length | Sampling rate (Hz) |
|---|---|---|---|---|
| Europe - Country 1 | 22 MCI 31 Control | 64 | 20 sec | 500 |

(continued)

| Location / database | Subjects | Number of EEG channels | Signal length | Sampling rate (Hz) |
|---|---|---|---|---|
| Asia - Country 1 | 22 MCI 23 AD 38 Control | 21 | 20 sec | 200 |
| Europe - Country 2 | 5 AD 5 Control | 19 | 20 sec | 128 |
| Europe - Country 3 | 8 AD 3 Control | 22 | 10-20 sec | 512 |
| Asia-Country 2 | 70 AD | 30 | 4 sec | 1000 |
| America - Country 1 | 57 Control | 19 | 5 min | 250 |
| Oceania - Country 1 | 30 Control | 19 | 10 min | 250 |

### *Neuromarkers identification*

The main steps necessary to extract relevant neuromarkers are:

- Standardization of the database whenever necessary in order to cancel out heterogeneity in data collection process (sampling rate, electrode number and location);
- Optional correction or removal of known artifacts; in particular, specific artifacts that are well characterized and can be corrected (e.g. eye blinks);
- Detection of remaining artefacts in order to discard parts of the signal that are not prone to signal analysis;
- Extraction of features from EEG time series, which can be spectral, Riemannian, complexity, topological, information driven amongst other;
- Modeling using machine learning technique to relate the features to the outcome using adequate cross-validation procedure.

### *Pre-processing*

As presented in the previous section, this study was based on the analysis of heterogeneous datasets, using different hardware settings, electrode placement, sampling rate and signal length. In order to homogenize all the databases and to allow a comparison between the different datasets, several pre-processing steps are applied:

1. First, EEGs signals were resampled at 128 Hz, in order to define a common temporal reference.
2. Second, signals were filtered with a band-pass filter in the frequency range 1-45 Hz and band-stop (notch) filtered in the frequency range 48-52 Hz or 58-62 Hz using 4th-order Butterworth filters in order to remove the noise induced by electrical power lines. These last notch filters were chosen depending on geographical location.
3. Finally, a common average referencing and spatial reconstruction was performed using a 4th-order spline. These transformations define a common spatial reference, which facilitates the comparisons between signals acquired with different headset on different electrodes location. The spatial reference for this work was the following set of electrodes in the International 10-20 system: Fp1, Fp2, F7, F3, Fz, F4, F8, T3, C3, Cz, C4, T4, T5, P3, Pz, P4, T6, O1, O2.

Any clinical or research grade EEG database can conveniently be normalized using the aforementioned procedure.

### *Artefact rejection*

After pre-processing, clean EEG data was modeled with a multidimensional Riemannian geometry model named: "Riemannian potato field", according to the following procedure:

Signals were band filtered using a bank of 5th-order Butterworth filters in five frequency bands: 2-6.5 Hz, 12-25 Hz, 25-34 Hz, 34-45 Hz, and 45-60 Hz;

Then, signals were segmented into overlapping epochs of 2 seconds every 250 ms, and represented by their spatial covariance matrix C;

Covariance matrices were then aggregated into a single averaged matrix per subject $\overline{C}$, which was assumed to represent a clean signal. The average matrix was calculated as a geometric mean in a Riemannian manifold;

Riemannian distances $d_R(C, \overline{C})$ between the covariance matrix C of each epoch and the subject-wise average $\overline{C}$ was computed;

For each recording, the distribution of distances was normalized and a metric of statistical significance was chosen to reject artefactual epochs. In particular, epochs whose covariance matrix had a z-score larger than 3 were rejected, e.g. when the difference between the observed distance and the mean distance is larger than three times the standard deviation.

After pre-processing and artifact rejection, signals are processed to extract two sets of features: geometric (i.e. Riemannian) distances to reference matrices and spectral densities.

***Feature extraction***

Riemannian distances

The Riemannian distances were defined as the distance (on Riemannian geometry) between a covariance matrix and another reference covariance matrix. It is possible to use several reference matrices. In this study, three reference covariance matrices were used, one for each group (AD, MCI, control), resulting in three neuromarkers.

The procedure to calculate these neuromarkers is the following:

1. Signals cleared from artefactual epochs were filtered with a bank of 5th-order Butterworth filters in four frequency ranges: delta (1-3 Hz), theta (3-6.5 Hz), alpha (6.5-12 Hz), beta (12-30 Hz), and then segmented in 2 second long epochs every 250 ms. These frequency bands were chosen in consideration of reported general slowing of EEG rhythms in elderlies. The result of this step is a spatio-frequential signal of 76 channels, corresponding to the number of electrodes (19) multiplied by the number of frequency bands (4);

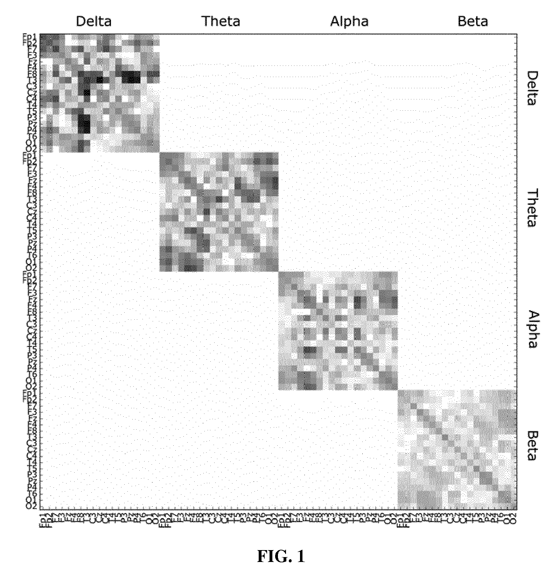

2. For each epoch, a covariance matrix is calculated, normalized by its determinant. Indeed, covariance matrices from different hardware settings can be very different and must be normalized to a common space. An example of such covariance matrix is illustrated in Figure 1;

3. Covariance matrices of each epoch are combined using a subject-wise geometric mean (in the Riemannian manifold), resulting in one average covariance matrix per subject;

4. For each reference group, the matrices of all subject of said group are combined using a geometric mean (in the Riemannian manifold), resulting in one reference covariance matrix. Other Riemannian clustering methods that combine several covariance matrices into one reference matrix can be used as well, such as mean-shift, k-means or principal geodesic analysis;

5. For each epoch, the Riemannian distance (in the Riemannian manifold) is calculated between the covariance matrix of said epoch and each of the reference covariance matrices. This results in one value per epoch and reference matrix. These values are aggregated per subject using a geometric mean (in the Euclidean space), reducing the result to one value per reference matrix.

Spectral densities

Spectral densities were extracted from the spectral densities of each EEG channel in several frequency bands.

To calculate these neuromarkers, the procedure is the following:

1. The power of signals cleared from artefactual epochs are calculated using Welch's method, using 2 seconds long epochs with 250 ms of overlap. This classic method estimates the signal power for each frequency of the Fourier representation of the epoched signal. Therefore, this step results in a vector of values per electrode and subject;

2. The powers estimated in the previous section are averaged across the frequencies of four ranges: delta (1-3 Hz), theta (3-6.5 Hz), alpha (6.5-12 Hz), and beta (12-30 Hz). This results in one average value per electrode and frequency band.

Multivariate analysis and feature selection

For this study, a total of 76 spectral densities (from 19 electrodes multiplied by 4 frequency bands) and 3 Riemannian distances (from 3 reference groups) were extracted, totaling 79 features. A logarithm function was applied to all features and then standardized by removing the mean and scaling by their variance.

A machine learning algorithm was employed to create a model that classifies the subject's class from the combination of 79 features. The model used was a least absolute shrinkage and selection operator (LASSO) general linear model with cross-validation. Such combination is achieved by means of a regularized linear model and is a mere illustration of the modeling technique that can be used.

The LASSO model computes the probability of a subject to belong to a class (e.g. the probability of a subject to have AD) as the following logistic function:

$$p(x \in \mathrm{AD} \,|\, w, w_0) = \frac{1}{1 + \exp(-(x^T w + w_0))} \,,$$

where x is a vector of the normalized neuromarkers of a subject. The model coefficients are represented by the vector $w$ and a bias term $w_0$. The range of this logistic function is real open interval between 0 and 1. Therefore, to classify a subject as Alzheimer's disease, a threshold value can be inferred from the prediction distribution (subject to optimization). Thus, when the estimated probability is over said threshold, the subject is considered to have Alzheimer's disease.

For the case of LASSO models, the estimation of the model coefficients (i.e. model fitting, or model training) is performed by solving the minimization problem

$$\min_{w, w_0} \lambda \|w\|_1 + \sum_{i=1}^{n} \log(\exp(-y_i(X_i^T w + w_0)) + 1) \,,$$

where $X$ is a matrix of all neuromarkers for each subject, and y is the class of each observation (for this particular representation of the minimization problem, y=1 is used for AD, y=-1 for control; MCI patients are not included in the training phase). Regularized models such as LASSO, are characterized by its regularization parameter lambda ($\lambda$), which must be calibrated, as it prevents overfitting and permits feature selection.

For the multivariate analysis of this work, the LASSO model was employed as follows:

1. First, the regularization parameter $\lambda$ was calibrated using a standard leave-one-out (LOO) cross validation procedure:

i. A fixed range of $\lambda$ values is defined;
ii. For each $\lambda$, the model is trained using all data (i.e. the neuromarkers from each subject) with the exception of one subject;
iii. The prediction error is measured on the removed subject;
iv. The previous two steps are repeated for all subjects and the mean error is calculated for each $\lambda$;
v. The optimal $\lambda$ is then used for all future model estimations;

2. Once the $\lambda$ parameter is fixed, the model is trained again with a selected subset or all subjects. Due to the regularization of the LASSO model, the trained model will have a only a subset of coefficients with non-zero values. Since each coefficient corresponds to one input feature, a non-zero coefficient indicates a selected feature for the model and it represents some important information needed to discriminate a subject from one class or another;
3. With a subset of features selected by the LASSO model, it is possible to determine if each feature is statistically significant. In other words, to estimate if the selection and value of its coefficient may be attributed to chance. In this study, a Wald's test was used for this task, giving a p-value for each coefficient. P-values under 0.05, 0.01 and 0.001 were considered as slightly significant, significant and very significant, respectively. Significance tests are not restricted to Wald's test, as is not the only statistical test that may be applied to identify significant features. Other well-known techniques can be applied, such as permutation tests, bootstrapping, or specific tests related to the underlying model (in this case, significance tests for LASSO - Lockhart R et al., 2014. A significance test for the lasso. Annals of statistics, 42(2), p.413).

Results of the multivariate analysis

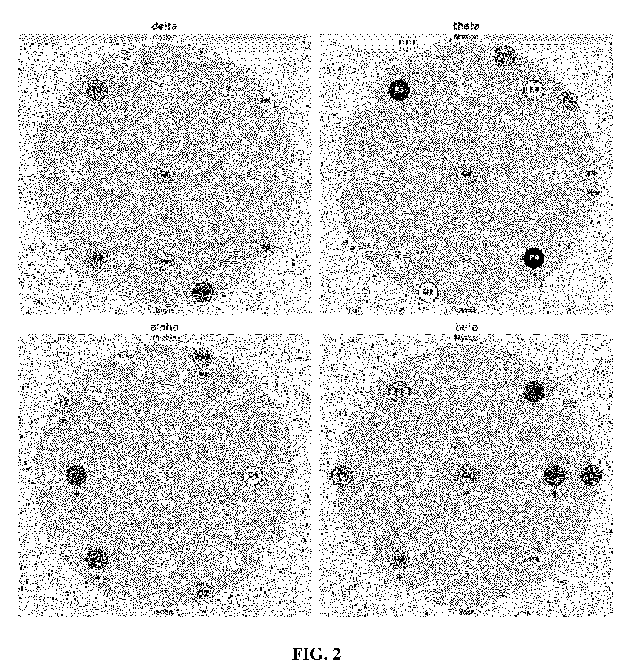

Cross-validation of the LASSO model returned values of $\lambda$ between 0.001 and 0.006, allowing for the selection of 32 predictive variables out of 79 features. These variables are displayed on topographic views in Figure 2.

Theses variables are the following: the three Riemannian distances and the spectral power densities for alpha frequency range for Fp2, F7, C3, C4, P3 and O2 electrodes; the spectral power densities for theta frequency range for Fp2, F3, F4, F8, Cz, T4, P4 and O1 electrodes, the spectral power densities for beta frequency range for F3, F4, T3, Cz, C4, T4, P3 and P4 electrodes, and the spectral power densities for delta frequency range for F3, F8, Cz, P3, Pz, T6 and O2 electrodes.

Significance test results are also shown in Figure 2 for spectral features (+: slightly significant, *: significant, ** or more: very significant). All three Riemannian distance features were found to be very significant.

Importance of geometric distances for neuromarkers modeling

The importance of Riemannian distances in the prediction of Alzheimer's disease was evidenced by the fact that all three features were selected by the LASSO model, and by their strong significance. Moreover, an improvement of the model predictive performance was assessed by repeating the multivariate analysis with two additional models that use a subset of the features: a model with only spectral features and a model with only Riemannian distances features. For each case, the model was trained with all the subjects except the ones from one testing database, while the model performance was evaluated by the AUROC of the prediction of testing database subjects. Performance measures of the models with different feature set are summarized in Table 2. For each database tested, the AUROC for the model that uses Riemannian distances and spectral features is at least 5% better than models with less features.

| Feature set | Training database | Testing database | Number of features selected | Sensitivity | Specificity | Accuracy | AUROC |
|---|---|---|---|---|---|---|---|
| Distances and Spectral features | All | All | 32 | 95.09 | 95.19 | 99.17 | 99.169 |
| | All except Asia–Country 1 | Asia – Country 1 | 31 | 76.32 | 78.69 | 80.21 | 80.206 |
| | All except Europe–Country 3 | Europe – Country 3 | 31 | 100.00 | 100.00 | 100.00 | 100.000 |
| | All except Europe–Country 2 | Europe – Country 2 | 35 | 100.00 | 100.00 | 100.00 | 100.000 |
| Distance features only | All | All | 3 | 90.80 | 86.30 | 92.64 | 92.638 |
| | All except Asia–Country 1 | Asia – Country 1 | 3 | 84.21 | 67.21 | 58.24 | 58.238 |
| | All except Europe–Country 3 | Europe – Country 3 | 3 | 100.00 | 90.91 | 91.67 | 91.667 |
| | All except Europe–Country 2 | Europe – Country 2 | 3 | 60.00 | 60.00 | 60.00 | 60.000 |
| Spectral features only | All | All | 28 | 90.18 | 90.74 | 97.03 | 97.030 |
| | All except Asia–Country 1 | Asia – Country 1 | 27 | 73.68 | 72.13 | 74.71 | 74.714 |
| | All except Europe–Country 3 | Europe – Country 3 | 26 | 100.00 | 100.00 | 100.00 | 100.000 |
| | All except Europe–Country 2 | Europe – Country 2 | 28 | 100.00 | 90.00 | 96.00 | 96.000 |

**Table 2: Comparison of model performances with different feature sets**

*Conclusion*

The neuromarkers identification procedure describes how a heterogeneous dataset of EEG signals was processed in order to extract a set of 79 features for each subject. Using a machine learning classification algorithm, such as a regularized generalized linear model, it was possible to select a subset of 32 features that can discriminate whether a subject has Alzheimer's disease or not. Statistical tests indicated that the neuromarkers related to Riemannian distances are very significant, while several (more than three) spectral density features were also significant.

**Alzheimer's disease *diagnosis***

Using a model that classifies the patient class from a reduced set of neuromarkers, as presented in the Neuromarkers identification section, a probability that a new subject has Alzheimer's disease is calculated. If this probability exceeds a diagnostic cut-off, the subject is considered to have Alzheimer's disease. In this section, it is explained how a new subject can be diagnosed for Alzheimer's disease using a selection of EEG neuromarkers determined in the multivariate analysis of the previous section.

*Diagnosis of a new subject*

The pre-conditions for the diagnosis of a new subject is that a model has been calculated from an EEG database as explained in the previous section. Therefore, a selection of neuromarkers with associated coefficients is known.
Assume a new subject whose condition regarding Alzheimer's disease is unknown. The following procedure will determine its probability to be ill:

1. Perform an EEG recording with an EEG headset, typically for at least 1 minute of clinical or research grade EEG under Eyes Closed condition;
2. Artefact removal and artefact detection (if required);
3. EEG signals standardizations: This step needs to transform the data to a spatial and temporal reference that is the same as the reference of the data used for the model;
4. Extract features from pre-processed and artifact-free EEG signals;
5. Pass these extracted features down to the model to calculate a probability estimate that indicates how likely is this EEG segment to belong to that of a patient with AD;
6. At this point an additional step can be performed to transform the probability into a binary response (ill or healthy). It is necessary to set a threshold value that will set the cutoff point of the model response. Therefore, if the probability calculated by the model exceeds the threshold value, the subject will be considered ill. Otherwise, the subject is considered healthy.

The score or the binary decision can be used alone or combined with other biomarkers for a variety of applications.

*Model generalization*

An additional analysis was performed to determine if the model structure used in this study can generalize and predict correctly new, unobserved subjects. This analysis is not necessary for the diagnostic of a new subject, but is presented here to prove the efficacy of the prediction procedure explained before. For this matter, a leave-one-out cross-validation strategy was used, where a subset of the data is purposely removed from the training procedure, in order to simulate the use-case where new unobserved data is to be evaluated.
The datasets presented in Table 1 were partitioned three times, resulting in three folds. Each fold consists in a different training and test sets, as shown in Table 3. Not all databases are included as a test set since they do not present enough patients to test both healthy and ill subjects.

**Table 3: Cross-validation folds details**

| Database | 1st fold | 2nd fold | 3rd fold |
|---|---|---|---|
| America - Country 1 | Train | Train | Train |
| Oceania - Country 1 | Train | Train | Train |
| Europe - Country 1 | Train | Train | Train |

(continued)

| Database | 1st fold | 2nd fold | 3rd fold |
|---|---|---|---|
| Japan - Country 2 | Train | Train | Train |
| Japan - Country 1 | Train | Train | Test |
| Europe - Country 2 | Train | Test | Train |
| Europe - Country 3 | Test | Train | Train |

For each fold the following procedure was applied:

1. Neuromarkers are recalculated for the training and testing set.
2. The model is fitted as explained in the multivariate analysis section.
3. The model is used to calculate the probability of each subject in the training set to be in the AD class, as explained in the previous section.
4. Different threshold values between 0 and 1 are tried. For each threshold value, a predicted class is determined for each subject in the training test. Since the real class of the subjects is known, correct predictions (true positives and true negatives), incorrect predictions (false positives and false negatives) and model performance measures are calculated, including sensitivity (probability of a positive test given that the patient is ill), specificity (probability of a negative test given that the patient is healthy) and accuracy (probability of true positive outcome and true negative outcome).
5. Compute a ROC curve, which summarizes the model performance as the threshold is varied. From all evaluated thresholds, an optimal cutoff point is selected as the threshold with the highest sensitivity and specificity.

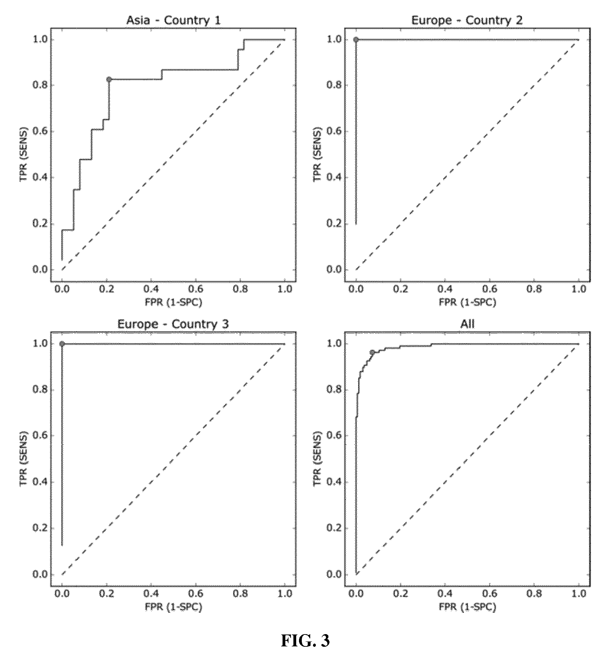

Model performance measures for each fold are summarized in Table 4. As a reference, a model with all subjects of all databases was also evaluated. To compare the efficacy of the models of each fold, the area under the ROC (AUROC) was used as a performance value (50% is the worst classifier, 100% is a perfect classifier). ROC curves are presented in Figure 3. The AUROC of the model for each fold reached high performances, between 80 and 100%. The AUROC of the model with all data is 99%, with an optimal cutoff of 0.37. In all cases, the specificity and sensitivity also reached satisfactory values, of at least 76%.

| Training database | Testing Database | Number of subjects | Optimal threshold | Sensitivity | Specificity | Accuracy | AUROC |
|---|---|---|---|---|---|---|---|
| All | All | 270 | 0.37 | 95.33 | 95.09 | 95.19 | 99.169 |
| All except Asia–Country 1 | Asia – Country 1 | 61 | 0.01 | 82.61 | 76.32 | 78.69 | 80.206 |
| All except Europe–Country 3 | Europe – Country 3 | 11 | 0.04 | 100.00 | 100.00 | 100.00 | 100.000 |
| All except Europe–Country 2 | Europe – Country 2 | 10 | 0.97 | 100.00 | 100.00 | 100.00 | 100.000 |

**Table 4: Cross-validated model performance for each fold**

*Conclusion*

The diagnosis procedure describes how the model estimated in the neuromarkers identification section can be used to calculate a probability of a subject to have AD, and to determine a clear yes-no diagnostic of AD when a threshold value that is fixed or optimized. Using different partitions of training and testing databases, it was possible to show that the diagnostic of AD subjects with a model based on EEG neuromarkers has a good predictive performance on unobserved data.

The diagnosis procedure is closely related to the assessment of a patient's condition: after an initial EEG recording, the technique analyses the recording and gives a score that indicates the probability a patient has to belong to a given diagnosis group (i.e. AD).

*Neuromarkers for monitoring applications*

The assessment technique presented in the previous sections can serve for the purpose of monitoring and also provides neuromarkers for neurofeedback applications.

Monitoring of condition progression is used following a recording at the clinician or at home at given times; in this case, the patient is equipped with a device composed of an EEG (headset and signal amplifier) connected to a computer that analyses the data and computes a score as explained in the previous sections. Then it either stores it and/or transmits it electronically for further analysis. Such home-use scenario offers the advantage of not requiring the intervention of a train specialist (for body fluid samples and analysis) nor the use of expensive machinery (MRI, CT, PET). The evolution of said predictive neuromarkers for the progression of the disease could be used to follow the efficacy of a treatment either to improve its design during a development or clinical research phase or for its titration during home delivery. Such treatment could be a drug, any type of intervention presumed to affect the CNS, or any neuromodulation technique delivered in the home or in the clinic for instance such as transcranial direct current stimulation (tDCS), repeated (or not) transcranial magnetic stimulation (TMS), neurofeedback, transcranial ultrasound stimulation, or electrocompulsive therapy (ECT). For instance, a company evaluating a tDCS protocol for the treatment of MCI patients could optimize the tDCS parameters (amplitude, frequency, duty cycle) based on real time progression of said neuromarkers.

*Neuromarker for therapeutic Neurofeedback*

Neurofeedback is used by patients diagnosed with AD. The subject is equipped with an EEG headset connected to a signal amplifier connected to a computer running a software that extracts the neuromarkers in real time after adequate pre-processing of the data (including artefact correction and detection). The said neuromarkers is then incorporated in a serious game environment, which is modulated in real time by the similarity with the subject's instantaneous EEG activity to that of a diseased patient - or in other term how likely the instantaneous EEG activity of the patient is to belong to the diseased group. The subject is instructed to play game several times a week for a typical length of 30 minutes and is rewarded by how much he/she can bend his/her EEG activity toward that of a normal population.

**Claims**

1. Predictive neuromarkers of Alzheimer's disease comprising:

   - at least one spectral feature obtained from EEG signals of a subject; and
   - at least one Riemannian distance between a spatiofrequential covariance matrix computed from the EEG signals of said subject and at least one reference spatiofrequential covariance matrix.

2. The predictive neuromarkers of Alzheimer's disease according to claim 1, wherein the at least one spectral feature is selected from the spectral power densities for alpha, beta, theta, gamma and delta frequency ranges for electrodes Fp1; Fp2; F7; F3; Fz; F4; F8; T3; C3; Cz; C4; T4; T5; P3; Pz; P4; T6; O1 and O2 according to the international 10-20 system.

3. The predictive neuromarkers of Alzheimer's disease according to claim 1 or claim 2, wherein the at least one spectral feature is selected from the spectral power densities for alpha frequency range for Fp2, F7, C3, C4, P3 and O2 electrodes; the spectral power densities for theta frequency range for Fp2, F3, F4,F8, Cz, T4, P4 and O1 electrodes, the spectral power densities for beta frequency range for F3, F4, T3, Cz, C4, T4, P3 and P4 electrodes, and the spectral power densities for delta frequency range for F3, F8, Cz, P3, Pz, T6 and O2 electrodes.

4. The predictive neuromarkers of Alzheimer's disease according to anyone of claims 1 to 3, wherein the at least one spectral features comprises the spectral power density for alpha frequency range for Fp2 electrode; the spectral power density for theta frequency range for P4 electrode and the spectral power density for alpha frequency range for 02 electrode.

5. The predictive neuromarkers of Alzheimer's disease according to anyone of claims 1 to 4, wherein the at least one Riemannian distance comprises the Riemannian distance between the spatiofrequential covariance matrix computed from the EEG signals of said subject and at least one reference spatiofrequential covariance matrix characteristics of a population of Alzheimer subjects.

6. The predictive neuromarkers of Alzheimer's disease according to claim 5, wherein the at least one Riemannian distance comprises:

- the Riemannian distance between the spatiofrequential covariance matrix computed from the EEG signals of said subject and at least one reference spatiofrequential covariance matrix characteristics of a population of Alzheimer subjects;
- the Riemannian distance between the spatiofrequential covariance matrix computed from the EEG signals of said subject and a reference spatiofrequential covariance matrix characteristics a control population which does not suffer from Alzheimer's disease or mild cognitive impairment; and
- the Riemannian distance the spatiofrequential covariance matrix computed from the EEG signals of said subject and at least one reference spatiofrequential covariance matrix characteristics of a population of mild cognitive impairment subjects.

7. The predictive neuromarkers of Alzheimer's disease according to claim 5 or claim **6,** wherein the at least one reference spatiofrequential covariance matrix characteristics of a population of Alzheimer subjects, the at least one reference spatiofrequential covariance matrix characteristics of a control population and/or the at least one reference spatiofrequential covariance matrix characteristics of a population of mild cognitive impairment subjects is obtained by a Riemannian clustering method from spatiofrequential covariance matrices of EEG signals of respectively a population of Alzheimer subjects, a control population and/or a population of mild cognitive impairment subjects.

8. The predictive neuromarkers of Alzheimer's disease according to anyone of claims **1** to **7,** further comprising at least one biomarker.

9. A non-invasive method of diagnosing the presence of Alzheimer's disease in a subject, comprising:

1) computing the predictive neuromarkers of Alzheimer's disease from EEG signals of said subject according to anyone of claims **1** to **8;** and
2) combining said predictive neuromarkers in a mathematic function to obtain a score useful for diagnosing the presence of Alzheimer's disease in said subject.

10. The non-invasive method according to claim **9,** further comprising:

3) diagnosing the presence or absence of Alzheimer's disease in said subject if the score is respectively below of above a diagnostic cut-off.

11. The non-invasive method according to claim **9** or claim **10,** wherein the mathematic function is a logistic function, preferably computed as follows:

$$p(x \in \mathrm{AD} \,|\, w, w_0) = \frac{1}{1 + \exp(-(x^T w + w_0))} ,$$

wherein x is a vector of the spectral features or the Riemannian distances, w is the vector of the coefficients and $w_0$ is a bias term.

12. A method for self-paced modulation of EEG signals of a subject in order to alleviate symptoms of Alzheimer's disease, said method comprising continuously:

- acquiring EEG signals from the subject; and
- computing the predictive neuromarkers of Alzheimer's disease from EEG signals of said subject according to anyone of claims **1** to **8;**
- computing a score from the predictive neuromarkers; and
- reporting the score to the subject.

13. A method for external modulation of EEG signals of a subject in order to alleviate symptoms of Alzheimer's disease, said method comprising continuously

- acquiring EEG signals from the subject; and
- computing the predictive neuromarkers of Alzheimer's disease from EEG signals of said subject according to anyone of claims **1** to **8;**
- computing a score from the predictive neuromarkers; and
- applying external modulation to the subject in order to modulate the score.

14. The method for self-paced modulation according to claim **12,** or the method for external modulation according to claim **13,** wherein the score is obtained by combining the predictive neuromarkers in a mathematic function, preferably a logistic regression.

15. A system for self-paced modulation or external modulation of EEG signals of a subject comprising:

- acquisition means for acquiring EEG signal from a subject;
- computing device for computing the predictive neuromarkers of Alzheimer's disease from EEG signals of said subject according to anyone of claims **1** to **8** and for computing a score from the predictive neuromarkers; and
- output means for reporting the score to the subject using a metaphor.

**FIG. 1**

**FIG. 2**

**FIG. 3**

**EP 3 241 489 A1**

<table>
<tr><td colspan="2" style="text-align:center">Europäisches Patentamt<br>European Patent Office<br>Office européen des brevets</td><td style="text-align:center">**EUROPEAN SEARCH REPORT**</td><td>**Application Number**<br>EP 16 16 8344</td></tr>
</table>

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2010/147913 A1 (BRAIN COMP INTERFACE LLC [US]; SIMON ADAM JAY [US]; VERMA AJAY [US]) 23 December 2010 (2010-12-23) * the whole document * | 9-11 | INV.<br>A61B5/00<br>A61B5/0476<br>A61B5/0482 |
| Y | HYUNJIN PARK: "Comparison of distance measures for manifold learning: Application to Alzheimer's brain scans", JOURNAL OF THE KOREAN PHYSICAL SOCIETY, vol. 61, no. 7, 1 October 2012 (2012-10-01), pages 1148-1155, XP055318276, KR ISSN: 0374-4884, DOI: 10.3938/jkps.61.1148 * the whole document * | 9-11 | ADD.<br>A61B5/048 |
| Y | WO 2013/164462 A1 (UNIV PARIS CURIE [FR]; CENTRE NAT RECH SCIENT [FR]; FOND ICM INST DU C) 7 November 2013 (2013-11-07) * the whole document * | 9-11 | |
| A | US 2015/038869 A1 (SIMON ADAM J [US] ET AL) 5 February 2015 (2015-02-05) * the whole document * | 9-11 | TECHNICAL FIELDS SEARCHED (IPC)<br><br>A61B |
| A,D | YILI LI ET AL: "Riemannian Distances for Signal Classification by Power Spectral Density", IEEE JOURNAL OF SELECTED TOPICS IN SIGNAL PROCESSING, IEEE, US, vol. 7, no. 4, 1 August 2013 (2013-08-01), pages 655-669, XP011520408, ISSN: 1932-4553, DOI: 10.1109/JSTSP.2013.2260320 * the whole document * | 9-11 | |

The present search report has been drawn up for all claims

| Place of search | Date of completion of the search | Examiner |
|---|---|---|
| The Hague | 10 November 2016 | Dhervé, Gwenaëlle |

## ANNEX TO THE EUROPEAN SEARCH REPORT
## ON EUROPEAN PATENT APPLICATION NO.

EP 16 16 8344

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2016

| Patent document cited in search report | | Publication date | Patent family member(s) | | Publication date |
|---|---|---|---|---|---|
| WO 2010147913 | A1 | 23-12-2010 | CA | 2765500 A1 | 23-12-2010 |
| | | | EP | 2442714 A1 | 25-04-2012 |
| | | | EP | 2682053 A1 | 08-01-2014 |
| | | | US | 2012150545 A1 | 14-06-2012 |
| | | | WO | 2010147913 A1 | 23-12-2010 |
| WO 2013164462 | A1 | 07-11-2013 | CA | 2872061 A1 | 07-11-2013 |
| | | | EP | 2844139 A1 | 11-03-2015 |
| | | | ES | 2588837 T3 | 07-11-2016 |
| | | | FR | 2990124 A1 | 08-11-2013 |
| | | | JP | 5952490 B2 | 13-07-2016 |
| | | | JP | 2015520627 A | 23-07-2015 |
| | | | US | 2015119745 A1 | 30-04-2015 |
| | | | WO | 2013164462 A1 | 07-11-2013 |
| US 2015038869 | A1 | 05-02-2015 | AU | 2012284246 A1 | 20-02-2014 |
| | | | CA | 2842027 A1 | 24-01-2013 |
| | | | US | 2015038869 A1 | 05-02-2015 |
| | | | WO | 2013012739 A1 | 24-01-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BHAT et al.** Clinical Neurophysiological and Automated EEG-Based Diagnosis of the Alzheimer's Disease. *Eur Neurol,* 2015
- **VIALATTE ; GALLEGO.** A Theta-band EEG based Index for Early diagnosis of Alzheimer's disease. *Journal of Alzheimer's disease,* 2014
- **LIZIO et al.** Electroencephalographic Rhythms in Alzheimer's Disease. *International Journal of Alzheimer's Disease,* 2011
- **DEURSEN et al.** Increased EEG gamma band activity in Alzheimer's disease and mild cognitive impairment. *J Neural Transm,* 2008
- **BARACHANT A.** Commande robuste d'un effecteur par une interface cerveau-machine EEG asynchrone. *PhD. Thesis,* 2012
- **LI Y ; WONG KM.** Riemannian Distances for Signal Classification by Power Spectral Density. *IEEE Journal of selected topics in signal processing,* August 2013, vol. 7 (4
- **FÖRSTNER W ; MOONEN B.** A metric for covariance matrices. *Quo vadis geodesia,* 1999, 113-128
- A significance test for the lasso. **LOCKHART R et al.** Annals of statistics. 2014, vol. 42, 413